# EUROPEAN PATENT APPLICATION

(11) **EP 4 623 995 A1**
(43) Date of publication of application: **01.10.2025**
(21) Application number: 25164401.9
(22) Date of filing: 18.03.2025
(51) Int. Cl.: A61P 35/00, C07K 16/18

(54) **MONOCLONAL ANTIBODY, REAGENT FOR MEASURING CRTAC1B, REAGENT KIT, AND METHOD FOR MEASURING CRTAC1B**

(30) Priority: 21.03.2024 JP 2024045431
(71) Applicant: SYSMEX CORPORATION, Kobe-shi Hyogo 651-0073 (JP); Public University Corporation Yokohama City University, Yokohama-shi, Kanagawa 236-0027 (JP)
(72) Inventor: Suganuma, Masatoshi, Kobe-shi, Hyogo 651-0073 (JP); Kubo, Hiroyuki, Kobe-shi, Hyogo 651-0073 (JP); Takei, Kohtaro, Yokohama-shi, Kanagawa 236-0004 (JP); Takahashi, Keita, Yokohama-shi, Kanagawa 236-0004 (JP)
(74) Representative: Hoffmann Eitle

(57) **Abstract**

[Problem] It is an object of the present invention to provide a monoclonal antibody that specifically binds to Crtac1B and/or a fragment thereof, a reagent and a reagent kit for measuring Crtac1B containing the antibody, and a method for measuring Crtac1B using the antibody.

[Solution means] The above problem is solved by an isolated monoclonal antibody, wherein the heavy chain comprises a CDR1 comprising the amino acid sequence of SEQ ID NO: 1, a CDR2 comprising the amino acid sequence of SEQ ID NO: 2, and a CDR3 comprising the amino acid sequence of SEQ ID NO: 3, and the light chain comprises a CDR1 comprising the amino acid sequence of SEQ ID NO: 4, a CDR2 comprising the amino acid sequence KAS, and a CDR3 comprising the amino acid sequence of SEQ ID NO: 5.

## Description

### [Technical field]

The present disclosure relates to a monoclonal antibody that specifically binds to cartilage acidic protein 1B (Cartilage acidic protein-1B:Crtac1B) and fragments thereof. The present invention relates to a reagent for measuring Crtac1B. The present invention relates to a reagent kit for measuring Crtac1B. The present invention relates to a method for measuring Crtac1B.

### BACKGROUND

Crtac1B, also known as lateral olfactory usher substance (LOTUS), is known to promote nerve regeneration by binding to Nogo receptors-1 and paired immunogloblin-like receptor-B expressed in the central nervous system and functioning as an antagonist towards to these receptors. Patent Document 1 describes that a protein level of Crtac1B was significantly reduced in cerebrospinal fluid (CSF) collected from a patient with an inflammation or a neurological disease accompanied by demyelination such as multiple sclerosis and optic neuromyelitis. Patent Document 1 describes that an anti-Crtac1B antibody that recognizes a region from position 516 to position 546 of an amino acid sequence of Crtac1B was prepared, and the protein level of Crtac1B in CSF was measured by immunoblotting analysis using the antibody.

### PRIOR ART

### PATENT DOCUMENT

[Patent document 1] U.S. Pat. No. 10,088,486

### [Summary of the invention]

### PROBLEM TO BE SOLVED BY THE INVENTION

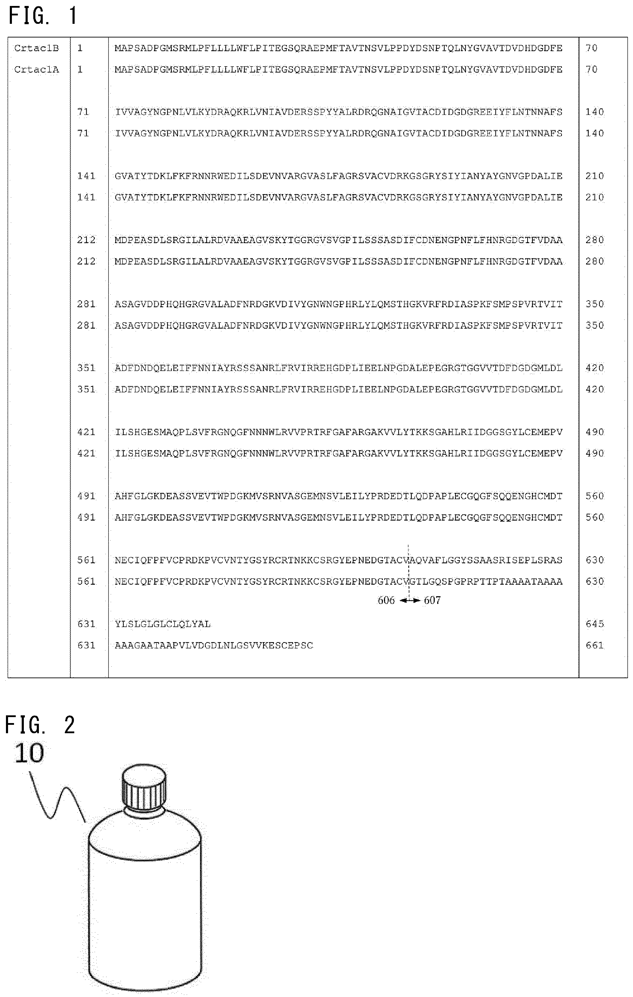

The Crtac1B protein is encoded by the Crtac1 gene, and from the gene, two variants of Crtac1A and Crtac1B are generated by alternative splicing. Referring to FIG. 1, amino acid sequences of human Crtac1B and Crtac1A are the same from position 1 to position 606 and differ from position 607 or later. To date, the present inventors have confirmed that not only a full length Crtac1B but also a fragment of Crtac1B is present in blood. In addition, as a fragment of Crtac1B, it was found that a relatively large number of proteins lacking a region from any one of amino acid residues at positions 610 to 612 of the amino acid sequence of Crtac1B to the amino acid residue at the C-terminus exist. On the other hand, Crtac1A is also present in a large amount in blood. Accordingly, it is an object of the present invention to provide a monoclonal antibody that specifically binds to Crtac1B and a fragment thereof. It is another object of the present invention to provide a reagent for measuring Crtac1B and a reagent kit containing the antibody, and a method for measuring Crtac1B using the antibody.

### MEANS FOR SOLVING THE PROBLEM

Therefore, the following inventions [1] to [10] are provided.
[1] An isolated monoclonal antibody comprising a heavy chain and a light chain, wherein the heavy chain comprises a CDR1 comprising the amino acid sequence of SEQ ID NO: 1, a CDR2 comprising the amino acid sequence of SEQ ID NO: 2, and a CDR3 comprising the amino acid sequence of SEQ ID NO: 3, and the light chain comprises a CDR1 comprising the amino acid sequence of SEQ ID NO: 4, a CDR2 comprising the amino acid sequence KAS, and a CDR3 comprising the amino acid sequence of SEQ ID NO: 5.
[2] The monoclonal antibody according to [1], wherein the heavy chain comprises a variable region comprising the amino acid sequence of SEQ ID NO: 6.
[3] The monoclonal antibody according to [1] or [2], wherein the light chain comprises a variable region comprising the amino acid sequence of SEQ ID NO: 7.
[4] The monoclonal antibody according to any one of [1] to [3], which specifically binds to Crtac1B and a fragment thereof.
[5] Wherein the Crtac1B is a protein consisting of the amino acid sequence of SEQ ID NO: 8, and the fragment is a protein: comprising at least a region consisting of an amino acid residue from the N-terminus to the position 609 of the amino acid sequence of SEQ ID NO: 8; and lacking a region from any one of the amino acid residues from the position 610 to the C-terminus of the amino acid sequence of SEQ ID NO: 8.
[6] A reagent for measuring Crtac1B, comprising the monoclonal antibody according to any one of [1] to [5].
[7] A reagent kit for measuring Crtac1B, comprising a first reagent comprising a capture body and a second reagent comprising a detection body, wherein the capture body or the detection body is the monoclonal antibody according to any one of [1] to [5].
[8] A method for measuring Crtac1B, comprising the steps of forming on a solid phase a complex comprising Crtac1B and/or a fragment thereof in a sample and a capture body, and detecting Crtac1B and/or the fragment thereof contained in the complex, wherein the capture body is the monoclonal antibody according to any one of [1] to [5].
[9] A method for measuring Crtac1B, comprising the steps of forming on a solid phase a complex comprising Crtac1B and/or a fragment thereof in a sample, a capture body, and a detection body, and detecting Crtac1B and/or the fragment thereof based on the detection body, wherein the capture body or the detection body is the monoclonal antibody according to any one of [1] to [5].
[10] The method according to any one of [8] and [9], wherein the sample is blood, plasma, serum, or cerebrospinal fluid.

### EFFECT OF THE INVENTION

According to the present invention, a monoclonal antibody that specifically binds to Crtac1B and a fragment thereof, a reagent and a reagent kit for measuring Crtac1B containing the antibody, and a method for measuring Crtac1B using the antibody are provided.

### BRIEF DESCRIPTION OF THE DRAWINGS

[Fig.1] Fig.1 illustrates a comparison of amino acid sequence of human Crtac1B (SEQ ID NO: 8) and amino acid sequence of human Crtac1A (SEQ ID NO: 30).
[Fig.2] Fig.2 illustrates a schematic diagram showing an example of the reagent of the present embodiment.
[Fig.3A] Fig.3A illustrates a schematic diagram showing an example of the reagent kit of the present embodiment.
[Fig.3B] Fig.3B illustrates a schematic diagram showing an example of the reagent kit of the present embodiment.
[Fig.4] Fig.4 illustrates a graph showing the results of measuring seven recombinant Crtac1B fragments by ELISA using monoclonal antibodies derived from hybridomas of clones 1B4 and 15C2.
[Fig.5] Fig.5 illustrates the results of analyzing the mixture of recombinant Crtac1A and Crtac1B and the two CSFs by immunoblotting using monoclonal antibodies derived from hybridomas of clones 1B4 and 15C2.
[Fig.6] Fig.6 illustrates the results of analyzing CSF and serum by immunoprecipitation and immunoblotting using monoclonal antibodies derived from hybridomas of clones 1B4 and 15C2.
[Fig.7] Fig.6 illustrates a graph showing the results of measuring a calibrator containing recombinant Crtac1B by sandwich ELISA using a monoclonal antibody derived from a hybridoma of 15C2 and a conventional antibody.

### DETAILED DESCRIPTION OF THE PREFERRED EMBODIMENTS

### [1. Monoclonal antibody]

The monoclonal antibody of the present embodiment (also referred to as "antibody of the present embodiment") is an isolated monoclonal antibody having a heavy chain and a light chain and includes three complementarity determining regions (CDRs) in each of variable regions of the heavy chain and the light chain. The three CDRs are called CDR1, CDR2 and CDR3, counting from the N-terminus of the antibody chain. The amino acid sequences of CDRs of the antibody of the present embodiment are as follows.
· Heavy chain CDR1:GYTFTDYN (SEQ ID NO: 1)
· Heavy chain CDR2:INPNYDSS (SEQ ID NO: 2)
· Heavy chain CDR3:TRSGGTY (SEQ ID NO: 3)
· Light chain CDR1:QNINVW (SEQ ID NO: 4)
· Light chain CDR2: KAS
· Light chain CDR3:QQAQSYPRT (SEQ ID NO: 5)

Preferably, the heavy chain of the antibody of the present embodiment includes a variable region comprising the amino acid sequence of SEQ ID NO: 6. Preferably, the light chain of the antibody of the present embodiment includes a variable region comprising the amino acid sequence of SEQ ID NO: 7. More preferably, the antibody of the present embodiment has a heavy chain comprising a variable region comprising the amino acid sequence of SEQ ID NO: 6 and a light chain comprising a variable region comprising the amino acid sequence of SEQ ID NO: 7. The amino acid sequence of the variable region of the antibody of the present embodiment is as follows.
· Heavy chain variable region 
EVQLQQFGAELVKPGASVKISCKASGYTFTDYNMDWVKQSHGKSLEWIGDINP
NYDSSSYNQKFKGKATLTVDKSSSTAYMELRSLTSEDTAVYYCTRSGGTYWG
QGTLVTVSA (SEQ ID NO: 6)
· Light chain variable region

DIQMNQSPSSLSASLGDTITITCHASQNINVWLSWYQQKPGNIPKLLIYKASNLH
TGVPSRFSGSGSGTGFSLTISSLQPEDIATYYCQQAQSYPRTFGGGTKLEIK (SEQ
ID NO: 7)

In the art, the amino acid sequence of the CDR can be specified by a known database that determines the position and/or amino acid sequence of the CDR based on the amino acid sequence of the variable region of the antibody or the polynucleotide sequence encoding the amino acid sequence. Examples of such a database include VBASE2 (Retter I. et al., Nucleic Acids Res., 2005, vol.33, D 671-D674). The amino acid sequence of the CDR of the antibody of the present embodiment is a sequence specified by VBASE2.

The antibody of the present embodiment is an antibody that specifically binds to Crtac1B and a fragment thereof. Here, "specifically binds to Crtac1B and the fragment thereof" means that the antibody of the present embodiment shows higher binding to Crtac1B and the fragment thereof than to an antigen other than Crtac1B and the fragment thereof. The binding between the antibody and the antigen can be measured by a method known in the art. Examples of such a method include immunoassay, surface plasmon resonance analysis, and isothermal titration calorimetry analysis. The antibody of the present embodiment was prepared using an antigen peptide prepared based on the amino acid sequence of human Crtac1B as shown in Example 1 described later but can also bind to Crtac1B of an animal species other than human by cross-reactivity. Preferably, the antibody of the present embodiment specifically binds to human Crtac1B and a fragment thereof. The full-length human Crtac1B is a protein consisting of the amino acid sequence set forth in SEQ ID NO: 8.

Preferably, the fragment of human Crtac1B is a protein comprising at least a region consisting of an amino acid residue from the N-terminus to the position 609 of the amino acid sequence of SEQ ID NO: 8 and lacking a region from any one of the amino acid residues from the position 610 to the C-terminus (position 645) of the amino acid sequence of SEQ ID NO: 8. Here, a fragment of human Crtac1B of an arbitrary length is also referred to as "fCrtac1B (1-X)". "fCrtac1B(1-X)" means a fragment consisting of an amino acid sequence from position 1 (N-terminus) to position X among the amino acid sequences set forth in SEQ ID NO: 8. "X" is an arbitrary natural number of 2 to 644. A fragment of human Crtac1B capable of specifically binding to the antibody of the present embodiment is fCrtac1B (1-X) having X of 609 or more and 644 or less. Among fragments of these human Crtac1B, for example, fCrtac1B (1-609), fCrtac1B (1-610), fCrtac1B (1-611), fCrtac1B (1-612), fCrtac1B (1-613) and fCrtac1B (1-614) are preferable. These fragments of human Crtac1B have been found by the present inventors to be relatively abundant in blood.

The antibody of the present embodiment may show weak binding to fCrtac1B (1-608). "Weak binding" means that the antibody of the present embodiment has 1/5 or less binding than the binding of the full length human Crtac1B or fCrtac1B (1-609). As shown in Example 1 described later, in the measurement by ELISA, the binding between the antibody of the present embodiment and fCrtac1B (1-608) is about 1/10 as compared with the binding to fCrtac1B (1-609).

The antibody of the present embodiment does not substantially bind to fCrtac1B (1-X) in which X is 607 or less. "Substantially not bound" includes not only does not bind to the antibody of the present embodiment at all but also shows binding to an extent that does not affect the measurement result in an immunoassay using the antibody of the present embodiment. For example, the binding between the antibody of the present embodiment and fCrtac1B (1-X) having X of 607 or less is 1/50 or less, preferably 1/100 or less, and more preferably 1/150 or less, compared with the binding between the antibody of the present embodiment and full length human Crtac1B or fCrtac1B (1-609). As shown in Example 2 described later, in the measurement by ELISA, little signal due to the reaction of the antibody of the present embodiment with fCrtac1B (1-607) or fCrtac1B (1-606) was detected. That is, no binding was shown.

The antibody of the present embodiment specifically binds to Crtac1B and a fragment thereof, but does not substantially bind to Crtac1A. For example, the binding between the antibody of the present embodiment and Crtac1A is 1/50 or less, preferably 1/100 or less, and more preferably 1/150 or less, compared with the binding between the antibody of the present embodiment and full length human Crtac1B or fCrtac1B (1-609). As shown in Example 4 described later, in immunoprecipitation and immunoblotting analysis using the antibody of the present embodiment, Crtac1B in a sample could be detected, but Crtac1A was hardly detected.

The antibody of the present embodiment may be a humanized antibody having a heavy chain containing a CDR1 consisting of the amino acid sequence set forth in SEQ ID NO: 1, a CDR2 consisting of the amino acid sequence set forth in SEQ ID NO: 2, and a CDR3 consisting of the amino acid sequence set forth in SEQ ID NO: 3, and a light chain containing a CDR1 consisting of the amino acid sequence set forth in SEQ ID NO: 4, a CDR2 consisting of the amino acid sequence KAS, and a CDR3 consisting of the amino acid sequence set forth in SEQ ID NO: 5. The humanized antibody is an antibody obtained by transplanting (CDR grafting) a polynucleotide sequence encoding the CDR of a non-human-derived antibody into a human antibody gene by genetic recombination technology. The antibody of the present embodiment may be a chimeric antibody comprising a heavy chain variable region comprising the amino acid sequence of SEQ ID NO: 6 and a light chain variable region comprising the amino acid sequence of SEQ ID NO: 7. The chimeric antibody is an antibody in which a variable region of an antibody derived from a certain animal species and a constant region of an antibody derived from an animal species different from the animal species are linked.

Class of antibody of the present embodiment may be any of IgG, IgA, IgM, IgD and IgE, and is preferably IgG. A subclass of IgG is not particularly limited, and may be any of IgG1, IgG2, IgG3 and IgG4. As used herein, the "antibody" includes not only an immunoglobulin form but also an antigen-binding antibody fragment. Examples of such an antibody fragment include Fab, F(ab')2, Fab', Fv, Fd, domain antibody (dAb), single-chain antibody (scFv), and diabody.

The antibody of the present embodiment may be labeled with a labeling substance known in the art. Methods for labeling a labeling substance to an antibody are known in the art. A preferred labeling method is a method in which a labeling substance is covalently bound to an antibody. A commercially available labeling kit or a cross-linker may be used. The labeling substance is not particularly limited, and examples thereof include a substance having a specific binding partner and a substance involved in generation of a signal.

Examples of the substance having a specific binding partner include biotins, haptens, and oligonucleotides. "Biotin" includes biotin and analogs thereof. Examples of the analog of biotin include desthiobiotin and biotin. The biotins specifically bind to avidins. "avidins" include avidin and analogs thereof. Examples of the analog of avidin include streptavidin, an avidin-like protein derived from tamogitake (tamavidin (registered trademark)), bradavidin, and rizavidin. Examples of the hapten include 2,4-dinitrophenyl (DNP) haptens. A DNP hapten (DNP group) covalently bound to a protein specifically binds to an anti-DNP antibody. The oligonucleotide specifically binds to an oligonucleotide having a sequence complementary to the nucleotide sequence.

The substance involved in generating a signal is, for example, a substance which itself generates a signal (hereinafter also referred to as "signal generating substance") and a substance which catalyzes a reaction of other substances to generate a signal. Examples of the signal generating substance include fluorescent substances, compounds containing radioactive isotopes, color developing substances, and chemiluminescent substances. Examples of the fluorescent substances include fluorescent dyes such as fluorescein isothiocyanate (FITC), rhodamine and Alexa Fluor (registered trademark), and fluorescent proteins such as GFP. Examples of the compound containing a radioactive isotope include nucleic acids containing any of ¹²⁵ I, ¹⁴ C, ³² P, ^{99m} Tc, ²²⁵ Ac, and the like, saccharides, and oligopeptides. Examples of the color developing substances include metal colloids such as gold nanocolloid. Examples of the chemiluminescent substance include ruthenium pyridine complex and acridinium ester. Examples of the substance which catalyzes a reaction of other substances to generate a detectable signal include enzymes. Examples of the enzyme include alkaline phosphatase, peroxidase, β-galactosidase, glucosidase, polyphenol oxidase, tyrosinase, acid phosphatase, and luciferase.

The antibody of the present embodiment can be prepared by a known genetic engineering method. The antibody can be prepared by a genetic engineering method, for example, by a method using a host cell synthesis system, a cell-free protein synthesis system using artificial tRNA, or the like. When a host cell synthesis system is used, first, an isolated polynucleotide encoding the heavy chain of the antibody of the present embodiment and an isolated polynucleotide encoding the light chain of the antibody of the present embodiment are incorporated into a protein expression vector known in the art to prepare an expression vector. The polynucleotide encoding the heavy chain and the polynucleotide encoding the light chain may be incorporated into one expression vector or may be separately incorporated into two expression vectors. Next, a host cell is transformed or transfected with the prepared expression vector to obtain a cell containing an expression vector containing a gene encoding the antibody of the present embodiment. Then, the cell is cultured to express the antibody of the present embodiment, and then the antibody is recovered and purified by a method known in the art. The type of the protein expression vector is not particularly limited. For example, the vector can be appropriately selected from vectors known in the art such as plasmid vectors and viral vectors. The type of the host cell is not particularly limited. The host cell may be any of eukaryotic cells and prokaryotic cells. Examples of the host cell include mammalian cells, insect cells, plant cells, yeast, and E. coli.

When a cell-free protein synthesis system is used, the protein can be synthesized by adding an amino acid, an energy molecule (for example, ATP, GTP, and the like), an isolated polynucleotide encoding the heavy chain of the antibody of the present embodiment, an isolated polynucleotide encoding the light chain of the antibody of the present embodiment or the like to a cell extract containing a translation factor. As the cell extract containing a translation factor, for example, a cell extract of E. coli, yeast, rabbit reticulocyte, wheat germ, insect cell, mammalian culture cell or the like can be used.

A further embodiment is a reagent for measuring Crtac1B (also referred to as "reagent of the present embodiment") containing the antibody of the present embodiment. The reagent of the present embodiment can be used for an immunoassay for measuring Crtac1B and a fragment thereof in a sample. The type of immunoassay is not particularly limited. The immunoassay can be selected from known methods such as immunoblotting analysis, immunoprecipitation, enzyme-linked immunosorbant method (ELISA), latex immunonephelometry, and immunocomplex transfer method (see JP-A-1-254868).

The antibody of the present embodiment included in the reagent of the present embodiment is used as a capture body or a detection body in an immunoassay. Here, the "capture body" is a substance that specifically binds to a test substance, and is a substance immobilized on a solid phase. When the capture body and the test substance bind, the test substance is captured on the solid phase. The capture body may be previously immobilized on a solid phase. "Detection body" is a substance that specifically binds to a test substance and is a substance for providing a detectable signal via a labeling substance. The detection body is preferably preliminarily labeled with a substance involved in generation of a signal. The detection body is usually not immobilized on the solid phase.

The reagent of the present embodiment may be provided to a user by containing the antibody of the present embodiment in a container. Fig.2 shows an example of the reagent of the present embodiment. Referring to Fig.2, 10 denotes a container containing the reagent of the present embodiment. A form of the reagent is not particularly limited, and may be solid (for example, powder, crystal, freeze-dried product, or the like) or liquid (for example, solution, suspension, emulsion, or the like). When the reagent of the present embodiment is a liquid containing the antibody of the present embodiment, a solvent is not particularly limited as long as the antibody of the present embodiment can be stably stored. Examples of such a solvent include aqueous solvents such as water, physiological saline, phosphate buffered saline (PBS), tris buffered saline (TBS), and Good's buffer solution. Examples of the Good's buffer include MES, Bis-Tris, ADA, PIPES, Bis-Tris-Propane, ACES, MOPS, MOPSO, BES, TES, HEPES, HEPPS, Tricine, Tris, Bicine, TAPS.

The reagent of the present embodiment may contain known additives. Examples of the additive include protein stabilizers such as bovine serum albumin (BSA), preservatives such as sodium azide, and inorganic salts such as sodium chloride.

A further embodiment is a reagent kit for measuring Crtac1B (also referred to as "reagent kit of the present embodiment") containing a first reagent containing a capture body and a second reagent containing a detection body. The reagent kit of the present embodiment can be used for an immunoassay for measuring Crtac1B and a fragment thereof in a sample. In the reagent kit of the present embodiment, either the capture body or the detection body is the antibody of the present embodiment. For example, when the first reagent contains the antibody of the present embodiment as a capture body, the second reagent contains a reagent that specifically binds to Crtac1B and a fragment thereof and contains a substance different from the antibody of the present embodiment (also referred to as "Crtac1B binding substance") as a detection body. Alternatively, when the second reagent contains the antibody of the present embodiment as a detection body, the first reagent contains a reagent containing Crtac1B binding substance as a capture body.

The Crtac1B binding substance may be, for example, an antibody different from that of the antibody of the present embodiment, an aptamer, or the like. The Crtac1B binding substance preferably binds to a site different from a site to which the antibody of the present embodiment binds in Crtac1B and a fragment thereof. The Crtac1B binding substance may bind to any one of regions consisting of an amino acid residue from the N-terminus to the position 607 of the amino acid sequence set forth in SEQ ID NO: 8, for example. Alternatively, the Crtac1B binding substance may bind to both Crtac1B and Crtac1A. As the Crtac1B binding substance, a commercially available antibody such as an anti-rat LOTUS antibody (ITM, 45-12C) or an anti-hCrtac1 antibody (RD) may be used.

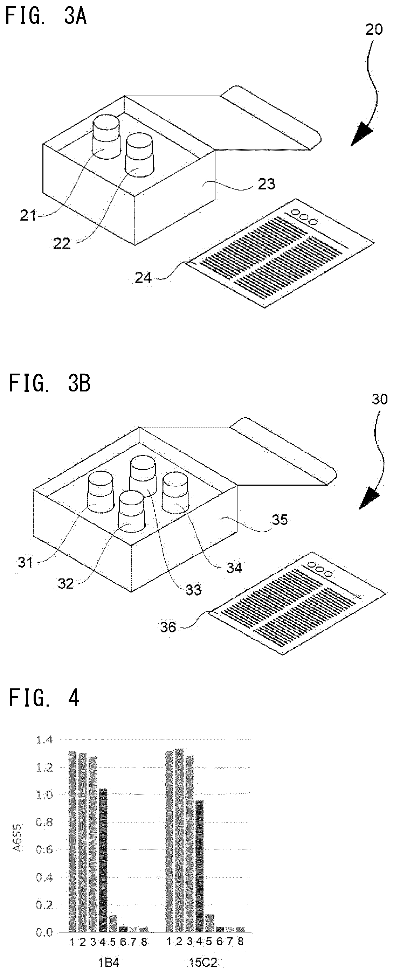

Fig.3A shows an example of the reagent kit of the present embodiment. In Fig.3A, 20 denotes a reagent kit, 21 denotes a first container containing a first reagent containing a capture body, 22 denotes a second container containing a second reagent containing a detection body, 23 denotes a packing box, and 24 denotes an attached document. Composition, usage, storage method and the like of each reagent may be described in the attached document. A form of each reagent in the reagent kit is not particularly limited, and may be solid (for example, powder, crystal, freeze-dried product, or the like) or liquid (for example, solution, suspension, emulsion, or the like). When each reagent of the reagent kit of the present embodiment is in a liquid form, the aqueous solvent described above can be used as a solvent. Each reagent in the reagent kit of the present embodiment may contain the above additive.

In the reagent kit of the present embodiment, the first reagent may include a capture body immobilized on a solid phase. Alternatively, the reagent kit of the present embodiment may further include a reagent containing a solid phase for immobilizing the capture body. The solid phase may be any insoluble carrier capable of immobilizing the capture body. The material of the solid phase is not particularly limited. For example, the material can be selected from organic polymer compounds, inorganic compounds, biopolymers, and the like. Examples of the organic polymer compound include latex, polystyrene, and polypropylene. Examples of the inorganic compound include magnetic bodies (iron oxide, chromium oxide, ferrite, and the like), silica, alumina, and glass. Examples of the biopolymer include insoluble agarose, insoluble dextran, gelatin, and cellulose. Two or more of these may be used in combination. The shape of the solid phase is not particularly limited, and examples thereof include microplates, particles, microtubes, test tubes, and membranes. Among them, microplates and particles (particularly, magnetic particles) are preferable.

The mode of immobilization of the capture body on the solid phase is not particularly limited. For example, the capture body and the solid phase may be directly bound, or the capture body and the solid phase may be indirectly bound via another substance. Examples of the direct binding between the solid phase and the capture body include adsorption or covalent binding to the solid phase surface by hydrophobic interaction. For example, when the solid phase is an ELISA microplate and the capture body is an antibody, the antibody is immobilized in the well of the plate by adsorption. Also, when the solid phase has a functional group on the surface, the capture body can be immobilized on the solid phase surface by covalent binding using a functional group. For example, when the solid phase is a particle having a carboxy group and the capture body is an antibody, the carboxy group on the particle surface is activated with WSC, and then reacted with NHS to form an NHS ester. Then, when the particle having an NHS ester and the antibody are contacted with each other, the NHS ester and the amino group of the antibody react with each other, and the antibody is covalently immobilized on the particle surface.

Examples of the indirect binding between the solid phase and the capture body include binding via a molecule that specifically binds to the capture body. By previously immobilizing such a molecule on the solid phase surface, the capture body can be immobilized on the solid phase. When the capture body is an antibody, for example, protein A, protein G, or the like can be used. In addition, the capture body can also be immobilized on the solid phase using a combination of substances intervening between the capture body and the solid phase. Examples of the combination of such substances include combinations of biotins and avidins, and haptens and anti-hapten antibodies. For example, when the capture body is labeled with biotins, the capture body can be immobilized on the solid phase by using a solid phase to which avidins are immobilized.

In the reagent kit of the present embodiment, the second reagent may contain a detection body labeled with the labeling substance. When the labeling substance is an enzyme, the reagent kit of the present embodiment may further include a reagent containing a substrate of the enzyme. The substrate can be appropriately selected from known substrates according to the type of enzyme. For example, when the enzyme is alkaline phosphatase, as a substrate,chemiluminescent substrates such as CDP-Star (registered trademark) (disodium 4-chloro-3-(methoxyspiro[1,2-dioxetane-3,2'-(5'-chloro)tricyclo[3.3.1.1^{3, 7}]decan]-4-yl)phenyl phosphate) and CSPD (registered trademark) (disodium 3-(4-methoxyspiro[1,2-dioxetane-3,2-(5'-chloro)tricyclo[3.1.1^{3,7}]decan]-4-yl)phenyl phosphate);Examples thereof include chromogenic substrates such as 5-bromo-4-chloro-3-indolyl phosphate (BCIP), disodium 5-bromo-6-chloro-indolyl phosphate, and p-nitrophenyl phosphate. When the enzyme is peroxidase, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB).

When the detection body contained in the second reagent is labeled with biotins, the reagent kit of the present embodiment may further include a reagent containing avidins labeled with a substance involved in generating a signal. By using these reagents, the detection body can be labeled with a substance involved in generating a signal via the bond between the biotins and the avidins.

When the detection body contained in the second reagent is an unlabeled antibody, the reagent kit of the present embodiment may further include a reagent containing an antibody labeled with a substance involved in generation of a signal and specifically binding to the detection body. In this case, the detection body is a primary antibody, and the labeled antibody that specifically binds to the detection body is a secondary antibody. When the substance involved in generating a signal is an enzyme, the reagent kit of the present embodiment may further include a reagent containing a substrate of the enzyme.

Fig.3B shows an example of the reagent kit of the present embodiment including a first reagent containing a capture body, a second reagent containing a detection body labeled with an enzyme, a third reagent containing a solid phase, and a fourth reagent containing a substrate of an enzyme. In Fig.3B, 30 denotes a reagent kit, 31 denotes a first container containing a first reagent containing a capture body, 32 denotes a second container containing a second reagent containing a detection body labeled with an enzyme, 33 denotes a third container containing a third reagent containing a solid phase, 34 denotes a fourth container containing a fourth reagent containing a substrate of an enzyme, 35 denotes a packing box, and 36 denotes an attached document. Composition, usage, storage method and the like of each reagent may be described in the attached document. A form of each reagent in the reagent kit is not particularly limited, and may be solid (for example, powder, crystal, freeze-dried product, or the like) or liquid (for example, solution, suspension, emulsion, or the like). When each reagent of the reagent kit of the present embodiment is in a liquid form, the aqueous solvent described above can be used as a solvent. Each reagent in the reagent kit of the present embodiment may contain the above additive.

The reagent kit of the present embodiment may further include a calibrator. The calibrator is a reagent containing a standard substance corresponding to Crtac1B or a fragment thereof at a predetermined concentration. Such a standard substance is preferably a polypeptide having all or a part of the amino acid sequence of Crtac1B and capable of binding a capture body and a detection body. Examples of the standard substance include a recombinant protein of full-length human Crtac1B or a fragment thereof, and a synthetic peptide having a part of the amino acid sequence of human Crtac1B. Examples of the recombinant protein of the fragment of human Crtac1B include a recombinant protein consisting of the amino acid sequence set forth in any one of SEQ ID NOs: 12 to 15. A synthetic peptide having a part of the amino acid sequence of human Crtac1B can be designed based on the amino acid sequence of SEQ ID NO: 8 when the site recognized by the capture body and the detection body is known.

The reagent kit of the present embodiment may include a plurality of sets of calibrators having different concentrations of standard substances from each other. The number of calibrators is not particularly limited, and can be selected from, for example, 2, 3, 4, 5, and 6 or more. The set of calibrators may include, as a negative control, an aqueous solvent containing no standard substance corresponding to Crtac1B or a fragment thereof. The concentration of the standard substance in each calibrator is not particularly limited and is preferably set so that a calibration curve for determining the concentration of Crtac1B and the fragment thereof in the sample can be prepared.

A further embodiment is a method for measuring Crtac1B using the antibody of the present embodiment (also referred to as "the method for measuring the present embodiment"). In the measurement method of the present embodiment, Crtac1B and/or a fragment thereof in a sample are measured by an immunoassay using the antibody of the present embodiment as a capture body or a detection body. The type of immunoassay is not particularly limited as long as it is a method using a capture body and a detection body. Examples thereof include a combination of immunoprecipitation and immunoblotting, sandwich ELISA, and immune complex transfer method.

The sample is not particularly limited as long as Crtac1B and/or a fragment thereof can be contained. A preferred sample is a biological sample. Examples of the biological sample include blood (whole blood), plasma, serum, cerebrospinal fluid, lymphatic fluid, tissue fluid, urine, and saliva. Among them, blood, plasma, serum and cerebrospinal fluid are preferable. When insoluble contaminants such as cells are contained in the sample, impurities may be removed from the sample by a known means such as centrifugal separation and filtration. The sample may be diluted with an appropriate aqueous medium as necessary. Such an aqueous medium is not particularly limited as long as it does not interfere with the measurement described later, and examples thereof include water, physiological saline, and a buffer solution. The buffer solution is as described above.

In the measurement method of the present embodiment, a complex containing Crtac1B and/or a fragment thereof in a sample and a capture body is formed on a solid phase. The complex can be formed on the solid phase by contacting the sample, the capture body, and the solid phase. Alternatively, the complex can be formed on the solid phase by contacting the sample with a capture body immobilized on the solid phase. The capture body and the solid phase are as described above.

Depending on the type of immunoassay, in the measurement method of the present embodiment, a complex containing Crtac1B and/or a fragment thereof in a sample, a capture body, and a detection body may be formed on a solid phase. The complex can be formed on the solid phase by contacting the sample, the capture body, the detection body, and the solid phase. Alternatively, the complex can be formed on the solid phase by contacting the sample, the capture body immobilized on the solid phase, and the detection body. The detection body is as described above.

In the measurement method of the present embodiment, Crtac1B and/or the fragment thereof contained in the complex can be detected using a detection body. The detection can be performed, for example, by liberating Crtac1B and/or the fragment thereof bound to the capture body on the solid phase from the capture body and binding the free Crtac1B and/or the fragment thereof to the detection body. In this case, the free Crtac1B and/or the fragment thereof may be captured on a new separate solid phase and then detected by a detection body. Alternatively, detection may be performed by further binding a detection body to Crtac1B and/or a fragment thereof bound to a capture body on a solid phase. When a complex containing Crtac1B and/or a fragment thereof, a capture body, and a detection body is formed on a solid phase, Crtac1B and/or the fragment thereof is detected based on the detection body contained in the complex.

Detection of Crtac1B and/or the fragment thereof by the detection body is preferably performed via a labeling substance. When the detection body is labeled with a substance involved in generating a signal as a labeling substance, the detection body is performed by detecting a signal generated by the substance in the detection body bound to Crtac1B and/or the fragment thereof. Also, when a secondary antibody against the detection body is used, a signal can be detected in the same manner to detect Crtac1B and/or a fragment thereof.

"Detecting a signal" herein includes qualitatively detecting the presence or absence of a signal, quantifying a signal intensity, and semi-quantitatively detecting the intensity of a signal. Semi-quantitative detection means to show the intensity of the signal in stages like "no signal generated", "weak", "medium", "strong", and the like. In the measurement method of the present embodiment, it is preferable to detect the intensity of a signal quantitatively or semi-quantitatively.

The method of detecting the signal can be selected from known methods according to the type of signal derived from the labeling substance contained in the detection body or the secondary antibody against the detection body. For example, when the labeling substance is an enzyme, signals such as light and color generated by reacting a substrate for the enzyme can be measured by using a known apparatus such as a spectrophotometer. When the labeling substance is a radioactive isotope, radiation as a signal can be measured using a known apparatus such as a scintillation counter. When the labeling substance is a fluorescent substance, fluorescence as a signal can be measured using a known apparatus such as a fluorescence microplate reader. The excitation wavelength and the fluorescence wavelength can be appropriately determined according to the type of fluorescent substance used.

The detection result of the signal can be used as the measurement result of Crtac1B and/or the fragment thereof. For example, when quantitatively detecting a signal intensity, a measured value of the signal intensity itself or a value acquired from the measured value can be used as the measured value of Crtac1B and/or the fragment thereof. Examples of the value acquired from the measured value of the signal intensity include a value obtained by subtracting the measured value of a negative control sample or the background value from the measured value. The measured value of the signal intensity may be applied to a calibration curve to determine the amount or concentration value of Crtac1B and/or the fragment thereof. The negative control sample can be appropriately selected, and examples thereof include a biological sample obtained from a healthy subject.

In the measurement method of the present embodiment, it is preferable to perform Bound/Free (B/F) separation for removing unreacted components between forming a complex and detecting Crtac1B and/or fragments thereof in the complex. The unreacted free component refers to a component not constituting a complex. Examples thereof include a capture body and a detection body not bound to Crtac1B and a fragment thereof. The means of B/F separation is not particularly limited. For example, when the solid phase is a particle, B/F separation can be performed by recovering only the solid phase capturing the complex by centrifugation. When the solid phase is a container such as a microplate or a microtube, B/F separation can be performed by removing a liquid containing an unreacted free component. In particular, when the solid phase is a magnetic particle, B/F separation can be performed by aspirating and removing a liquid containing an unreacted free component by a nozzle while magnetically constraining the magnetic particles with a magnet, which is preferable from the viewpoint of automation of measurement. After removing the unreacted free component, the solid phase capturing the complex may be washed with a suitable aqueous medium such as PBS.

The Crtac1B and/or the fragment thereof in the sample may be measured by a sandwich ELISA method using the capture body immobilized on the magnetic particles and the detection body of the present embodiment labeled with a labeling substance. In this case, the measurement may be performed using a commercially available fully automated immunoassay system such as the HISCL series (manufactured by Sysmex Corporation).

In the measurement method of the present embodiment, the concentration of Crtac1B and/or the fragment thereof in the sample may be acquired using the calibrator described above. In this case, the measurement method of the present embodiment may further include measuring the standard substance in the calibrator and acquiring the concentration of Crtac1B and/or the fragment thereof in the sample based on the measurement result of the standard substance. The calibrator and the standard substance are as described above.

The standard substance in the calibrator can be bound to the capture body and the detection body, so that the standard substance can be measured in the same manner as the Crtac1B and/or the fragment thereof in the sample. Since the concentration of the standard substance in the calibrator is known, the concentration of Crtac1B and/or the fragment thereof in the sample can be acquired from the measured value of Crtac1B and/or the fragment thereof in the sample based on the measured value and the concentration of the standard substance in the calibrator. Preferably, a calibration curve is prepared from the measured value and concentration of the standard substance in the calibrator, and the measured value of Crtac1B and/or the fragment thereof in the sample in the sample is applied to the calibration curve to obtain the concentration of Crtac1B and/or the fragment thereof in the sample in the sample. In preparing a calibration curve, for example, the measured values of the standard substances in a plurality of calibrators are plotted on an XY plane in which the concentration of the standard substance in the calibrator is taken on an X-axis and the measured values are taken on a Y-axis. Then, it can be prepared by obtaining a straight line or a curve by a known method such as a least squares method.

Hereinbelow, the present invention will be described in detail by examples, but the present invention is not limited to these examples.

### EXAMPLES

### Acquisition of monoclonal antibody

### (1) Design and preparation of antigen peptide

To obtain a monoclonal antibody that binds to Crtac1B but does not bind to Crtac1A, three peptides containing the 3 residues (AQV) at positions 607 to 609 of the amino acid sequence of human Crtac1B (SEQ ID NO: 8) on the C-terminal side were designed as antigen peptides (referred to as P1, P2 and P3, respectively). The amino acid sequences of each antigen peptide are shown in Table 1. Synthesis of these antigen peptides and conjugates with carrier proteins (keyhole limpet hemocyanin (KLH) or BSA) were commissioned to Scram Corporation.

**[Table 1]**

| **ANTIGEN PEPTIDE** | **AMINO ACID SEQUENCE** | **SEQ ID NO** |
|---|---|---|
| **P1** | **Ac-ADGTACVAQVAA-CONH₂** | **9** |
| **P2** | **Ac-CPNEDGTAAVAQVAA-CONH₂** | **10** |
| **P3** | **NH₂-PEG6-GCSRGYEPNEDGTACVAQVA-CONH₂** | **11** |

P1 was a linear peptide containing an amino acid sequence from position 601 to position 610 of the amino acid sequence set forth in SEQ ID NO: 8. P2 was a linear peptide containing a sequence in which the cysteine residue at position 605 was replaced with an alanine residue in the amino acid sequence from position 598 to position 610 of the amino acid sequence set forth in SEQ ID NO: 8. In P1 and P2, the N-terminus was acetylated, the C-terminus was amidated, and KLH was conjugated to a cysteine residue. P3 was a peptide comprising an amino acid sequence from position 592 to position 610 of the amino acid sequence set forth in SEQ ID NO: 8, wherein a disulfide bond was formed between the two cysteine residues. In P3, the C-terminus was amidated and KLH was conjugated to the N-terminal amino group.

### (2) Preparation of hybridoma

Hybridomas producing monoclonal antibodies were prepared by immunizing mice with antigen peptides P1, P2 and P3, respectively, by lymph node method. Specifically, an emulsion (antigen concentration: 1.0 mg/mL) in which each antigen peptide conjugated with KLH, and an adjuvant were mixed was injected into the ridge of the mouse. Inoculation of antigen peptides by injection was performed in 3 mice per antigen peptide. About 2 weeks after the first inoculation, the above emulsion was boosted by injecting the same mouse again. Furthermore, one week after the second inoculation, the final immunization was performed by injecting the emulsion into the same mouse again. Three days after the third inoculation, lymphocytes were isolated from lymph nodes of mice. The isolated lymphocytes were subjected to cell fusion with myeloma to obtain a hybridoma.

### (3) Primary screening

In order to select hybridomas producing monoclonal antibodies reactive to the antigen peptide, the culture supernatant of each hybridoma was measured by ELISA. In the primary screening, as a positive antigen, a peptide to which BSA was conjugated was used as a carrier protein for each of the antigen peptides P1, P2 and P3. Specific operations were as follows.

The positive antigen was diluted with PBS to a concentration of 1 µg/mL. Positive antigen (50 µL) was added to each well of a 96 well plate. Then, the plate was incubated at 4° C. for 17 hours, and a positive antigen was immobilized in the well to obtain an antigen immobilized plate. Each well was washed three times with PBS (250 µL). To each well was added a blocking buffer (250 µL, 0.5% skim milk/PBS) and blocked at room temperature for 1 hour. Culture supernatant (50 µL) of each hybridoma was added to each well, and the cells were incubated at room temperature for 1 hour. Each well was washed three times with PBS (250 µL). An ALP-labeled anti-mouse IgG goat antibody (SBI, product number 1030-04) was diluted 1:2,500 with PBS. A solution (50 µL) of this labeled antibody was added to each well, and the well was incubated at room temperature for 30 minutes. Each well was washed three times with PBS (250 µL). A chemiluminescent substrate solution (Kind-King modified) was added to each well at 100 µL, and the plate was gently shaken at room temperature. The plate was set in a microplate reader, and the emission intensity was measured for each well.

Based on the result of the primary screening, hybridomas (positive hybridomas) of culture supernatants reactive to the antigen peptide were selected from hybridomas derived from mice immunized with each antigen peptide. The number of selected positive hybridomas is shown in Table 2.

**[Table 2]**

| **ANTIGEN PEPTIDE** | **NUMBER OF POSITIVE HYBRIDOMA** |
|---|---|
| **P1** | **28** |
| **P2** | **39** |
| **P3** | **127** |

### (4) Secondary screening

Culture supernatant of hybridomas selected in primary screening was measured by ELISA, and secondary screening of hybridomas was performed. In the secondary screening, six recombinant Crtac1B fragments prepared by recombinant technology were used as positive antigens (referred to as #1, #2, #3, #4, #5 and #6, respectively). These positive antigens were recombinant proteins lacking a region from any one of amino acid residues 608 to 612 and 615 of the amino acid sequence of human Crtac1B (SEQ ID NO: 8) to the amino acid residue of the C-terminus. As a negative control, a recombinant protein lacking a region from the amino acid residue at position 607 to the amino acid residue at the C-terminus of the amino acid sequence of SEQ ID NO: 8 was used (referred to as #7). The amino acid sequences of each positive antigen and negative control are shown in SEQ ID NOs: 12 to 18. The amino acid sequences of the positive antigen and the negative control after position 594 are shown in Table 3 and SEQ ID NOs: 19 to 25.

**[Table 3]**

| **No.** | **AMINO ACID SEQUENCE FROM POSITION 594 OF CRTAC1B FRAGMENT** | **SEQ ID NO** | **TYPE** |
|---|---|---|---|
| **#1** | **--- RGYEPNEDGTACVAQVAFLGG** | **19** | **POSITIVE ANTIGEN** |
| **#2** | **--- RGYEPNEDGTACVAQVAF** | **20** | |
| **#3** | **--- RGYEPNEDGTACVAQVA** | **21** | |
| **#4** | **--- RGYEPNEDGTACVAQV** | **22** | |
| **#5** | **--- RGYEPNEDGTACVAQ** | **23** | |
| **#6** | **--- RGYEPNEDGTACVA** | **24** | |
| **#7** | **--- RGYEPNEDGTACV** | **25** | **NEGATIVE CONTROL** |

### (4.1) Preparation of recombinant Crtac1B fragment

Recombinant Crtac1B fragments of #1 to #7 were prepared as follows. First, a gene encoding the full length of Crtac1B was artificially synthesized. Using this synthetic gene as a template, a gene encoding each Crtac1B fragment was amplified by PCR. Using the obtained PCR product as an insert, the cDNA was incorporated into an Expi293 (registered trademark) expression plasmid pcDNA (registered trademark) 3.4-TOPO (registered trademark) to obtain an expression vector of a recombinant Crtac1B fragment. The obtained expression vectors were transfected into Expi293 cells using Expi293 (registered trademark) Expression System (Thermofisher Scientific). After culturing the transfected cells, the culture was collected, and the culture supernatant was acquired by centrifugation. The culture supernatant was dialyzed with 10 mM potassium phosphate buffer (pH 7.4). The dialyzed culture supernatant was applied to a hydroxyapatite column (Bio-Rad, Bio-Scale Mini CHT Type I Cartridge, 5mL). Proteins containing the recombinant Crtac1B fragment were separated and eluted by using a potassium phosphate buffer as an eluate and changing the concentration of the eluate from 10 mM to 500 mM. The fraction containing the recombinant Crtac1B fragment was collected and applied to a gel filtration column (GE Healthcare, HiLoad Superdex 200 26 600). The mobile phase (10 mM potassium phosphate buffer (pH 7.4), 150 mM NaCl) was flowed into the column, and the protein containing the recombinant Crtac1B fragment was separated and eluted. The fraction containing the recombinant Crtac1B fragment was collected and concentrated to about 1 mg/mL by an ultrafiltration membrane (Millipore, AmiconUltra-15). The molecular weight of each recombinant Crtac1B fragment was measured by LC-MS. The analysis conditions of LC-MS are shown below.

### <LC-MS analysis conditions>

Manufacturer: Waters Corporation
LC Type: ACQUITY UPLC (registered trademark) H-Class
MS type: Xevo (registered trademark) G2Q-TOFMS
Measuring principles: LC-ESI-Tof-MS
Columns: C4 BEH 300 C4 (1.7µm, 2.1 x 50mm)
Buffer (A): 0.1%(v/v) TFA, H2O
Buffer (B): 0.1% (v/v) TFA, acetonitrile

The theoretical values of molecular weight based on the amino acid sequence and the molecular weight determined by LC-MS are shown in Table 4. As can be seen from Table 4, for all recombinant Crtac1B fragments, the molecular weight determined by LC-MS was substantially the same as the theoretical value. Therefore, it was shown that each of the acquired recombinant Crtac1B fragments was a protein consisting of the amino acid sequences set forth in SEQ ID NOs: 12 to 18, respectively.

**[Table 4]**

| **No.** | **AMINO ACID SEQUENCE FROM POSITION 594 OF CRTAC1B FRAGMENT** | **MOLECULAR WEIGHT (THEORETICAL VALUE)** | **MOLECULAR WEIGHT (LC-MS)** | **DIFFERENCE** |
|---|---|---|---|---|
| **#1** | **--- RGYEPNEDGTACVAQVAFLGG** | **64155** | **64151** | **-4** |
| **#2** | **--- RGYEPNEDGTACVAQVAF** | **63928** | **63925** | **-3** |
| **#3** | **--- RGYEPNEDGTACVAQVA** | **63781** | **63776** | **-5** |
| **#4** | **--- RGYEPNEDGTACVAQV** | **63710** | **63707** | **-3** |
| **#5** | **--- RGYEPNEDGTACVAQ** | **63611** | **63607** | **-4** |
| **#6** | **--- RGYEPNEDGTACVA** | **63483** | **63481** | **-2** |
| **#7** | **--- RGYEPNEDGTACV** | **63412** | **63408** | **-4** |

### (4.2) Antigen immobilized ELISA

The positive antigen and the negative control were diluted to a concentration of 20 µg/mL with dilution buffer I (20 mM potassium phosphate buffer (pH 7.4), 150 mM NaCl). A positive antigen (50 µL) and a negative control (50 µL) were added to each well of a 96 well plate, respectively. Then, the plate was incubated at 4° C. overnight, and the positive antigen and the negative control were immobilized in the well to obtain an antigen immobilized plate. Each well was washed twice with a washing solution (250 µL, 0.05% Tween (registered trademark) 20/20 mM potassium phosphate buffer (pH 7.4), 150 mM NaCl). To each well was added a blocking buffer (250 µL, 5% skim milk/20 mM potassium phosphate buffer (pH 7.4), 150 mM NaCl), and blocked at room temperature for 90 minutes. Culture supernatant (50 µL) of each hybridoma selected by primary screening was added to each well, and the cells were incubated at 37° C. for 1 hour. Each well was washed three times with a washing solution (250 µL). The HRP-labeled anti-mouse IgG antibody (MBL, product number 330) was diluted 1: 1,000 with dilution buffer II (1% skim milk/20 mM potassium phosphate buffer (pH 7.4), 150 mM NaCl). A solution (50 µL) of this labeled antibody was added to each well, and the well was incubated at 37° C. for 1 hour. Each well was washed five times with a washing solution (250 µL). Chemical chromogenic substrate solutions (Bio-Rad, TMB Peroxidase EIA Substrate Kit) were added to respective wells at 50 µL, and the plate was allowed to stand at room temperature under shading for 30 minutes. The plate was set in a microplate reader (Thermo Fisher Scientific, Varioskan (registered trademark) Flash), and the color development intensity at 655 nm was measured for each well.

Based on the result of the secondary screening, hybridomas (positive hybridomas) of the culture supernatant reactive to the positive antigen were selected from hybridomas selected by the primary screening. The number of selected positive hybridomas is shown in Table 5. As shown in Table 5, a hybridoma producing a monoclonal antibody reactive to a positive antigen could not be obtained from a hybridoma derived from a mouse immunized with the antigen peptides P1 and P2. Two hybridomas producing monoclonal antibodies reactive to positive antigens were obtained from hybridomas derived from mice immunized with antigen peptide P3.

**[Table 5]**

| **ANTIGEN PEPTIDE** | **NUMBER OF POSITIVE HYBRIDOMA** |
|---|---|
| **P1** | **0** |
| **P2** | **0** |
| **P3** | **2** |

### (5) cloning

Two hybridomas selected by secondary screening were seeded by a limiting dilution method. Culture of each hybridoma was continued, and two hybridomas of a single clone of interest were acquired. Each clone was called "1B4" and "15C2".

### Confirmation of reactivity of monoclonal antibody to antigen

Reactivity of the monoclonal antibody contained in the culture supernatant of the hybridomas of clones 1B4 and 15C2 to the antigen was confirmed by ELISA using the 7 recombinant Crtac1B fragments described above. A specific operation was the same as that of the antigen immobilized ELISA method of Example 1. For comparison, ELISA was performed in the same manner using a 96 well plate in which no antigen was immobilized. The results are shown in Fig.4.

In Fig.4, graphs of "1" to "7" represent color development intensity when recombinant Crtac1B fragments of #1 to #7 were used as antigens, and a graph of "8" represents color development intensity when a plate not solidified with antigen was used. As can be seen from Fig.4, monoclonal antibodies produced from hybridomas of clones 1B4 and 15C2 reacted with recombinant Crtac1B fragments of #1 to #4, but reactivity of #5 to recombinant Crtac1B fragment was significantly reduced. From this, it was found that these monoclonal antibodies showed weak binding to the recombinant Crtac1B fragment of #5.
In addition, as to these monoclonal antibodies, the color development intensity when the recombinant Crtac1B fragments of #6 and #7 were used was substantially the same as that when a plate without immobilizing the antigen was used. From this, it was found that these monoclonal antibodies do not substantially bind to the recombinant Crtac1B fragment of #6 and #7. Therefore, it was suggested that the monoclonal antibody produced from the hybridoma of clones 1B4 and 15C2 recognizes a region on the C-terminal side including the 3 residues (AQV) at positions 607 to 609 of the amino acid sequence set forth in SEQ ID NO: 8.

### Study of specificity of monoclonal antibody to antigen (1)

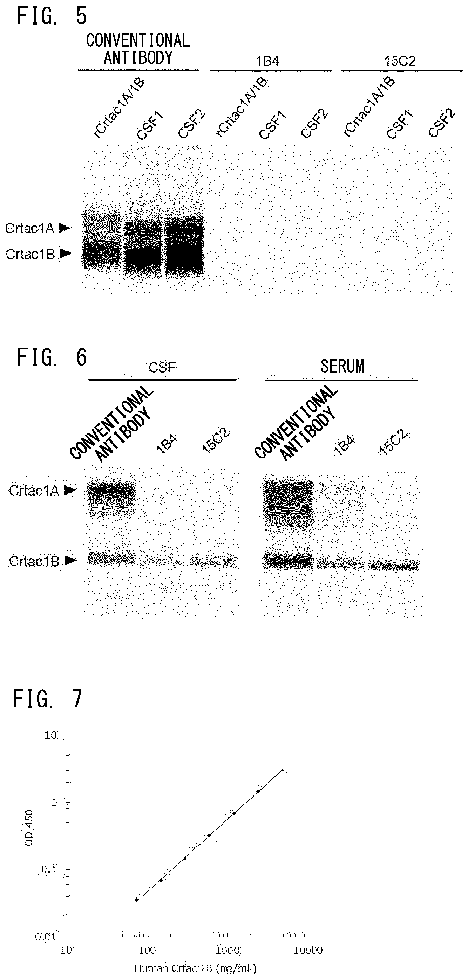

Immunoblotting (IB) analysis using a monoclonal antibody purified from the culture supernatant of hybridomas of clones 1B4 and 15C2 was performed on human cerebrospinal fluid (2 specimens: referred to as CSF1 and CSF2) by simple Western (registered trademark) system Wes (registered trademark) (Protein Simple Corporation). In the analysis by Wes, capillary electrophoresis and IB of the sample were performed fully automatically by the apparatus. Specific operations such as preparation of an electrophoresis sample, preparation of a diluent of a primary antibody, and analysis by an apparatus were performed according to the protocol attached to Wes. As a control sample, recombinant proteins of Crtac1A and Crtac1B were used. These recombinant proteins were prepared in the same manner as in Example 1. For comparison, analysis using an anti-rat LOTUS antibody (ITM, 45-12C) as a primary antibody was also performed. The results are shown in Fig.5.

In Fig.5, "conventional antibody" represents the anti-rat LOTUS antibody, and "rCrtac1A/1B" represents recombinant proteins of Crtac1A and Crtac1B. As can be seen from Fig.5, when a conventional antibody was used, bands of Crtac1A and Crtac1B were detected in a control sample and cerebrospinal fluid. However, when monoclonal antibodies derived from clones 1B4 and 15C2 were used, a band was not detected. In the electrophoresis sample, the protein in the sample was denatured by addition of a sample buffer and heating. Therefore, it was suggested that these monoclonal antibodies cannot recognize Crtac1B in which the structure of the protein was linearly changed and recognize the C-terminal conformation of Crtac1B.

### Study of specificity of monoclonal antibody to antigen (2)

For human CSF and human serum, immunoprecipitation using a monoclonal antibody purified from the culture supernatant of hybridomas of clones 1B4 and 15C2 was performed, and IB analysis by Wes was performed. For comparison, immunoprecipitation (IP) using an anti-hCrtac1 antibody (RD) and IB analysis using an anti-rat LOTUS antibody (ITM, 45-12C) were performed.

### (1) IP method

As a sample, CSF (300 µL) and serum (500 µL) were used per antibody. Each sample and protein G sepharose were mixed and rotated at 4° C. for 1 hour to remove contaminants that non-specifically bind to protein G from each sample in advance. Protein G solid phase-treated magnetic particles (60 µL, Veritas, Dynabeads (registered trademark) Protein G) were dispensed into 1.5-mL tubes, and washed twice with dilution buffer III (500 µL, 0.02% Tween (registered trademark) 20/PBS (pH 7.4)). Magnetic collection was performed to remove the supernatant, and each antibody (24 µg) and the above dilution buffer III (200 µL) were added to the magnetic particles. The tube was stirred at room temperature for 1 hour, and then collected to remove the supernatant. The magnetic particles were washed five times with the above dilution buffer III (300 µL), and then collected to remove supernatant. Each sample from which impurities were removed was added to the magnetic particles, and the tube was stirred at room temperature for 5 hours. The supernatant was collected by magnetic collection, and the magnetic particles were washed three times with the above dilution buffer III (300 µL). A 2× sample buffer (25 µL) was added to the magnetic particles, and the tube was stirred for 10 minutes. The supernatant was collected by magnetic collection into a new tube, and 2-mercaptoethanol (0.4 µL) was added thereto. The tube was heated at 100° C. for 7 minutes to prepare an electrophoretic sample. The 2x sample buffer was prepared by diluting a 5x sample buffer (255 mM Tris (pH 6.8), 50% glycerol, 5% SDS, 0.05% bromophenol blue) with water.

### (2) IB analysis

The prepared electrophoresis sample was subjected to IB analysis by Wes (Protein Simple Corporation). A specific operation was performed according to the protocol attached to Wes. As the primary antibody, monoclonal antibodies derived from clones 1B4 and 15C2 were used. For comparison, analysis using an anti-rat LOTUS antibody (ITM, 45-12C) was also performed. The results are shown in Fig.6. As can be seen from Fig.6, when IP and IB were performed using a conventional antibody, bands of Crtac1A and Crtac1B were detected in CSF and serum. On the other hand, when IP and IB were performed using monoclonal antibodies derived from clones 1B4 and 15C2, only a band of Crtac1B was detected in CSF and serum. That is, it was shown that the monoclonal antibodies derived from clones 1B4 and 15C2 do not substantially bind to Crtac1A and specifically bind to Crtac1B. Therefore, it was suggested that Crtac1B can be specifically detected from a biological sample such as CSF and serum by immunoassay using monoclonal antibodies derived from clones 1B4 and 15C2.

### Example 5 Analysis of amino acid sequence of variable region of monoclonal antibody (1) Sequence analysis

Total RNA was prepared from each hybridoma of clones 1B4 and 15C2. cDNA was synthesized using the prepared total RNA as a template. The cDNA was double-stranded with RNase H and ligated with adapters. Using the obtained cDNA as a template, PCR was performed using a primer of constant region of heavy chain and an adapter primer, and a primer of constant region of light chain and an adapter primer, to amplify a region encoding a variable region. PCR products of the heavy chain and the light chain were purified and cloned into a cloning vector. The obtained cloning vector was introduced into E. coli to obtain a transformant. A plasmid was prepared from the obtained transformant, and the polynucleotide sequence was analyzed. An amino acid sequence was determined based on the acquired polynucleotide sequence. Polynucleotide sequences of CDRs of the heavy chain and the light chain were identified from the acquired polynucleotide sequences by the integrated V gene database VBASE2. Amino acid sequences were determined based on the identified polynucleotide sequences.

### (2) Results

As a result of sequence analysis, the polynucleotide sequences of the heavy chain and the light chain of clone 1B4 were the same as the polynucleotide sequences of the heavy chain and the light chain of clone 15C2. That is, it was found that clones 1B4 and 15C2 were hybridomas having the same antibody gene. Hereinafter, the anti-Crtac1B antibody produced by these hybridomas will be referred to as "15C2 antibody". The 15C2 antibody was a mouse IgG1 antibody. Amino acid sequences of CDR1, CDR2 and CDR3 of the heavy chain and light chain of the 15C2 antibody were as follows.
- Heavy chain CDR1:GYTFTDYN (SEQ ID NO: 1)
- Heavy chain CDR2:INPNYDSS (SEQ ID NO: 2)
- Heavy chain CDR3:TRSGGTY (SEQ ID NO: 3)
- Light chain CDR1:QNINVW (SEQ ID NO: 4)
- Light chain CDR2: KAS
- Light chain CDR3:QQAQSYPRT (SEQ ID NO: 5)

The amino acid sequences of the variable regions of the heavy chain and the light chain of the 15C2 antibody were as follows.
• Heavy chain variable region 
EVQLQQFGAELVKPGASVKISCKASGYTFTDYNMDWVKQSHGKSLEWIGDINP
NYDSSSYNQKFKGKATLTVDKSSSTAYMELRSLTSEDTAVYYCTRSGGTYWG
QGTLVTVSA (SEQ ID NO: 6)
• Light chain variable region

DIQMNQSPSSLSASLGDTITITCHASQNINVWLSWYQQKPGNIPKLLIYKASNLH
TGVPSRFSGSGSGTGFSLTISSLQPEDIATYYCQQAQSYPRTFGGGTKLEIK (SEQ
ID NO: 7)

The amino acid sequences of the heavy chain and the light chain of the 15C2 antibody were as follows. The polynucleotide sequence encoding the heavy chain of the 15C2 antibody is set forth in SEQ ID NO: 26, and the polynucleotide sequence encoding the light chain is set forth in SEQ ID NO: 27.
• Heavy chain variable region 
MEWSWIFLFLLSGTAGVLSEVQLQQFGAELVKPGASVKISCKASGYTFTDYNM
DWVKQSHGKSLEWIGDINPNYDSSSYNQKFKGKATLTVDKSSSTAYMELRSLT
SEDTAVYYCTRSGGTYWGQGTLVTVSAAKTTPPSVYPLAPGSAAQTNSMVTL
GCLVKGYFPEPVTVTWNSGSLSSGVHTFPAVLQSDLYTLSSSVTVPSSTWPSET
VTCNVAHPASSTKVDKKIVPRDCGCKPCICTVPEVSSVFIFPPKPKDVLTITLTPK
VTCVVVDISKDDPEVQFSWFVDDVEVHTAQTQPREEQFNSTFRSVSELPIMHQD
WLNGKEFKCRVNSAAFPAPIEKTISKTKGRPKAPQVYTIPPPKEQMAKDKVSLT
CMITDFFPEDITVEWQWNGQPAENYKNTQPIMDTDGSYFVYSKLNVQKSNWE
AGNTFTCSVLHEGLHNHHTEKSLSHSPGK (SEQ ID NO: 28)
• Light chain variable region

MRVLAELLGLLLFCFLGVRCDIQMNQSPSSLSASLGDTITITCHASQNINVWLS
WYQQKPGNIPKLLIYKASNLHTGVPSRFSGSGSGTGFSLTISSLQPEDIATYYCQ
QAQSYPRTFGGGTKLEIKRADAAPTVSIFPPSSEQLTSGGASVVCFLNNFYPKDI
NVKWKIDGSERQNGVLNSWTDQDSKDSTYSMSSTLTLTKDEYERHNSYTCEA
THKTSTSPIVKSFNRNEC (SEQ ID NO: 29)

### Example 6 Measurement of Crtac1B using 15C2 antibody

Using the 15C2 antibody as a detection body, Crtac1B was measured by sandwich ELISA using an anti-rat LOTUS antibody (ITM, 45-12C) as a capture body.

### (1) Preparation of biotin-labeled 15C2 Fab'

The 15C2 antibody was pepsin-digested to prepare an F(ab') ₂. F(ab') The ₂ was reduced with 0.3 M 2-mercaptoethylamine solution and gel-filtered through Amicon Ultra-4, 30k (Merck) to obtain a reduced Fab'. The reduced Fab' and biotin-PEAC ₅ maleimide (Dojin Chemical Laboratory Co., Ltd.) were mixed and reacted at 4° C. overnight. The mixture was gel-filtered through Amicon Ultra-4, 30k (Merck) to obtain biotin-labeled 15C2 Fab'.

### (2) Preparation of a sample containing Crtac1B

Recombinant human Crtac1B was prepared in the same manner as in Example 1. A solution containing recombinant human Crtac1B measured based on absorbance at 280 nm using a spectrophotometer was diluted with a dilution buffer I (20 mM potassium phosphate buffer (pH 7.4), 150 mM NaCl) to prepare 7 samples having protein concentrations of 0.22, 0.45, 0.89, 1.79, 3.58, 7.15 and 14.3 ng/mL.

### (3) measurement

An anti-rat LOTUS antibody (ITM, 45-12C) was added to each well of a 96-well plate for ELISA to a concentration of 0.25 µg/well. Then, the plate was incubated at 25° C. overnight, and the antibody was immobilized in the well to obtain an antibody immobilized plate. Each well was washed three times with a washing solution (250 µL, 0.05% Tween (registered trademark) 20/20 mM potassium phosphate buffer (pH 7.4), 150 mM NaCl). Each sample (50 µL) was added to each well and incubated at 25° C. for 30 minutes. Each well was washed three times with a washing solution (250 µL). A 0.5 µg/mL biotin-labeled 15C2 Fab' solution (50 µL) was added to each well and incubated at 25° C. for 30 minutes. Each well was washed three times with a washing solution (250 µL). 1 µg/mL HRP-labeled streptavidin (50 µL) was added to each well and incubated at 25° C. for 15 minutes. Each well was washed three times with a washing solution (250 µL). Chemical chromogenic substrate solutions (Bio-Rad, TMB Peroxidase EIA Substrate Kit) were added to respective wells at 50 µL, and the plate was allowed to stand at room temperature under shading for 30 minutes. Each well was washed three times with a washing solution (250 µL), and 1 M H ₂ SO ₄ (100 µL) was added to each well to stop the reaction. The plate was set on a microplate reader (Thermo Fisher Scientific, Varioskan (registered trademark) Flash), and the intensity of color development was measured at 450 nm for each well. The results are shown in Table 6 and Fig.7.

**[Table 6]**

| | | | | | | | |
|---|---|---|---|---|---|---|---|
| **CONCENTRATION** (ng/mL) | 14.3 | 7.15 | 3.58 | 1.79 | 0.89 | 0.45 | 0.22 |
| **ABSORBANCE** (450 nm) | 2.99 | 1.451 | 0.684 | 0.319 | 0.146 | 0.069 | 0.036 |

As can be seen from Table 6, the detected signal intensity increased depending on the concentration of Crtac1B. Therefore, it was shown that Crtac1B can be measured by sandwich ELISA using the 15C2 antibody. In addition, it was shown that a calibration curve can be prepared as shown in Fig.7 by using a plurality of recombinant human Crtac1B solutions having different concentrations as calibrators.

### [Explanation of the numbers in the Drawings]

10: Container containing a reagent
20, 30: Reagent kit
21, 31: first container
22, 32: second container
23, 35: Packing box
24,36: Attached document
33: Third container
34: The fourth container

## Claims

1. An isolated monoclonal antibody comprising a heavy chain and a light chain,
wherein the heavy chain comprises a CDR1 comprising the amino acid sequence of SEQ ID NO: 1, a CDR2 comprising the amino acid sequence of SEQ ID NO: 2, and a CDR3 comprising the amino acid sequence of SEQ ID NO: 3; and
wherein the light chain comprises a CDR1 comprising the amino acid sequence of SEQ ID NO: 4, a CDR2 comprising the amino acid sequence KAS, and a CDR3 comprising the amino acid sequence of SEQ ID NO: 5.

2. The monoclonal antibody according to claim 1, wherein the heavy chain comprises a variable region comprising the amino acid sequence of SEQ ID NO: 6.

3. The monoclonal antibody according to claim 1, wherein the light chain comprises a variable region comprising the amino acid sequence of SEQ ID NO: 7.

4. The monoclonal antibody according to claim 1, which specifically binds to Crtac1B and a fragment thereof.

5. The monoclonal antibody according to claim 4,
wherein the Crtac1B is a protein consisting of the amino acid sequence of SEQ ID NO: 8, and
wherein the fragment is a protein: comprising at least a region consisting of an amino acid residue from the N-terminus to the position 609 of the amino acid sequence of SEQ ID NO: 8; and lacking a region from any one of the amino acid residues from the position 610 to the C-terminus of the amino acid sequence of SEQ ID NO: 8.

6. A reagent for measuring Crtac1B, comprising the monoclonal antibody according to any one of claims 1 to 5.

7. A reagent kit for measuring Crtac1B, comprising a first reagent comprising a capture body and a second reagent comprising a detection body, wherein the capture body or the detection body is the monoclonal antibody according to any one of claims 1 to 5.

8. A method for measuring Crtac1B, comprising the steps of
forming on a solid phase a complex comprising Crtac1B and/or a fragment thereof in a sample and a capture body, and
detecting Crtac1B and/or the fragment thereof contained in the complex,
wherein the capture body is the monoclonal antibody according to any one of claims 1 to 5.

9. The method according to claim 8, wherein the sample is blood, plasma, serum, or cerebrospinal fluid.

10. A method for measuring Crtac1B, comprising the steps of:
forming on a solid phase a complex comprising Crtac1B and/or fragments thereof in a sample, a capture body, and a detection body; and
detecting Crtac1B and / or the fragment thereof based on the detection body,
wherein the capture body or the detection body is the monoclonal antibody according to any one of claims 1 to 5.

11. The method according to claim 10, wherein the sample is blood, plasma, serum, or cerebrospinal fluid.
